# EUROPÄISCHE PATENTSCHRIFT

(11) **EP 3 204 325 B1**
(45) Veröffentlichungstag und Bekanntmachung des Hinweises auf die Patenterteilung: **26.09.2018**
(21) Anmeldenummer: 15787140.1
(22) Anmeldetag: 30.09.2015
(51) Int. Cl.: B67B 3/12

(54) **VERSCHLIESSWERKZEUG SOWIE ZIEHRING FÜR EIN VERSCHLIESSWERKZEUG**
CAPPING HEAD WITH DEFORMATION RING
TÊTE DE CAPSULAGE AVEC BAGUE DE DEFORMATION

(30) Priorität: 07.10.2014 DE 102014114561
(43) Veröffentlichungstag der Anmeldung: 16.08.2017
(73) Patentinhaber: KHS GmbH, 44143 Dortmund (DE)
(72) Erfinder: NAABER, Matthias, 55559 Bretzenheim (DE)
(86) Internationale Anmeldenummer: PCT/EP2015/072527
(87) Internationale Veröffentlichungsnummer: WO 2016/055323

(56) Entgegenhaltungen:
- DE-A1- 4 110 456
- DE-A1- 4 332 740
- DE-A1- 19 626 680
- GB-A- 770 745

## Beschreibung

Die Erfindung bezieht sich auf ein Verschließwerkzeug gemäß Oberbegriff Patentanspruch 1 sowie auf einen Verschließring entsprechend Oberbegriff Patentanspruch 14.

Verschließwerkzeuge sowie mit derartigen Werkzeugen versehene Verschließmaschinen zum Verschließen von Flaschen mit Kronenkorken oder dergleichen Verschlüssen sind bekannt (DE 39 18 504 A1, DE 196 26 680 A1). Die DE 196 26 680 A1 zeigt dabei ein Verschießwerkzeug gemäß dem Oberbegriff des Anspruchs 1. Das Verschließen der Flaschen mit den Kronenkorken erfolgt bei diesen Verschließwerkzeugen grundsätzlich derart, dass nach dem Aufsetzen eines Kronenkorkens auf die Mündung einer Flasche der Rand dieses Kronenkorkens durch Absenkens eines Verschließstempels mit einem dortigen Ziehring bleibend hinter einen Mündungswulst der Flasche verformt wird. Der Ziehring ist dabei in einer Ziehringaufnahme des Verschließstempels oder eines von diesem gebildeten Ziehringhalters angeordnet.

Weiterhin ist auch bekannt und auch erforderlich, insbesondere die Verschließwerkzeuge von Zeit zu Zeit, d.h. in vorgegebenen oder vorgewählten Zeitabständen zu reinigen und/oder zu desinfizieren, und zwar durch Behandlung bzw. Umspülen mit einem flüssigen und/oder gas- und/oder dampfförmigen Reinigungs- und/oder Desinfektionsmedium, beispielsweise im Rahmen einer CIP-Reinigung und/oder Desinfektion. Hierbei müssen insbesondere auch die mit den Verschlüssen in Berührung kommenden Flächen des jeweiligen Verschließwerkzeugs und dabei vor allem auch des dortigen Ziehrings behandelt werden, was bei bisher bekannten Verschließwerkzeugen allerdings im Wesentlichen nur an freiliegenden Flächen möglich ist.

Aufgabe der Erfindung ist es, ein Verschließwerkzeug aufzuzeigen, welches eine gegenüber bekannten Verschließwerkzeugen verbesserte Reinigung und/oder Desinfektion im Bereich des Ziehringhalters und des Ziehringes ermöglicht.

Zur Lösung dieser Aufgabe ist ein Verschließwerkzeug entsprechend dem Patentanspruch 1 ausgebildet. Ein Ziehring zur Verwendung bei dem Verschließwerkzeug ist Gegenstand des Patentanspruchs 14.

Der Erfindung liegt die Erkenntnis zugrunde, dass es für eine optimale Reinigung und/oder Desinfektion des Verschließwerkzeuges notwendig ist, nicht nur die freiliegenden Flächen, auch des Ziehringes mit dem Reinigungs- und/oder Desinfektionsmedium zu behandeln, sondern auch den Spalt oder Übergang zwischen dem Ziehringhalter und dem Ziehring, d.h. insbesondere die axialen und radialen Abstützbereiche der den Ziehring aufnehmenden Ziehringaufnahme sowie die gegen diese Abstützbereiche anliegenden Flächen des Ziehringes. Dies wird durch die erfindungsgemäße Ausbildung des Verschließwerkzeugs bei hoher Betriebssicherheit, d.h. insbesondere bei einer zuverlässigen Verankerung des Ziehringes am Ziehringhalter erreicht.

In Weiterbildung der Erfindung ist das Verschließwerkzeug beispielsweise so ausgeführt,
dass die Strömungswege jeweils durchgehend zwischen einer Unterseite und einer Oberseite des Ziehringhalters und des Ziehringes ausgebildet sind und sich beispielsweise bezogen auf die Werkzeugachse zumindest axial oder im Wesentlichen axial, bevorzugt auch zusätzlich radial oder im Wesentlichen radial und/oder tangential oder im Wesentlichen tangential erstrecken, oder
dass die Ziehringaufnahme oder die diese Aufnahme bildende Öffnung an ihrer Innenfläche und/oder der Ziehring an der sich am Ziehringhalter abstützenden Umfangs- und/oder Stirnfläche profiliert sind, und zwar jeweils zur Ausbildung einer Vielzahl von erhabenen Bereichen und angrenzenden, diese erhabenen Bereiche beispielsweise auch zumindest teilweise umschließenden Vertiefungen oder Gräben, und dass die erhabenen Bereiche in Summe die Anlagefläche der Ziehringaufnahme und/oder die Anlagefläche des Ziehrings bilden,
oder
dass das Flächenmaß der Summe der von den erhabenen Bereichen gebildeten Anlage- oder Abstützflächen für den Ziehring deutlich kleiner ist als das Flächenmaß, das nicht profilierte Anlage- oder Abstützflächen für den Ziehring in Summe aufweisen würden, beispielsweise um wenigstens 50%, oder
dass die Profilierung in Form von Nuten und dazwischen liegenden, die Anlage- oder Abstützflächen bildenden Stegen ausgeführt ist,
oder
dass die Profilierung für eine bezogen auf die Werkzeugachse axiale Abstützung des Ziehringes von einer Vielzahl von Vorsprüngen gebildet ist, die voneinander beabstandet und um die Werkzeugachse oder die Achse des Ziehringes verteilt in der Öffnung und/oder an einer Stirnfläche des Ziehringes vorgesehen sind,
oder
dass die die Profilierung bildenden Nuten mit ihrer Längserstreckung parallel oder im Wesentlichen parallel zur Werkzeugachse oder zu einer Achse des Ziehringes oder schräg oder schraubenförmig in Bezug auf diese Achsen ausgeführt sind,
oder
dass in den Stegen zusätzliche Nuten oder Vertiefungen vorgesehen sind, die jeweils beidendig in eine zwei Stege beabstandende Nut münden und beispielsweise bezogen auf die Maschinenachse oder bezogen auf die Achse des Ziehringes tangential oder im Wesentlichen tangential orientiert sind,
oder
dass die zur axialen Abstützung des Ziehringes dienenden Vorsprünge an jeweils einem Steg ausgebildet sind und vorzugsweise in einen gemeinsamen Vorsprung übergehen, der ringförmig ausgebildet und die Werkzeugachse umschließend in die die Ziehringaufnahme oder in die diese Aufnahme bildende Öffnung hineinreicht,
oder dass der Ziehring schwimmend, d.h. mit einem gewissen axialen und/oder radialen Spiel in der Ziehringaufnahme oder Öffnung des Ziehringhalters angeordnet ist,
oder
dass im Ziehringhalter wenigstens ein in den Spalt zwischen dem Ziehringhalter und benachbarten Flächen des Ziehringes mündender Kanal zum Zuführen und/oder Abführen des Behandlungsmediums vorgesehen ist, wobei der wenigstens eine Kanal vorzugsweise an einer Fläche des Ziehringhalters außerhalb der Ziehringaufnahme oder der diese Aufnahme bildenden Öffnung offen ist,
oder
dass der wenigstens eine Kanal in wenigstens eine in der Ziehringaufnahme oder Öffnung ausgebildete, die Werkzeugachse beispielsweise zumindest teilweise umschließende Nut mündet,
oder
dass zwei in Bezug auf die Werkzeugachse axial gegeneinander versetzte Nuten vorgesehen sind, in die jeweils wenigstens ein Kanal mündet und die strömungsmäßig miteinander verbunden sind,
oder
dass die wenigstens eine Nut seitlich begrenzende Stege die axialen sowie radialen Abstützbereiche des Ziehringes bilden, und dass dieser Abstützbereich bevorzugt die Profilierung aufweist,
wobei die vorgenannten Merkmale jeweils einzeln oder in beliebiger Kombination vorhanden sein können.

Der Ausdruck "im Wesentlichen" bzw. "etwa" bzw. "ca." bedeutet im Sinne der Erfindung Abweichungen vom jeweils exakten Wert um +/- 10%, bevorzugt um +/-5% und/oder Abweichungen in Form von für die Funktion unbedeutenden Änderungen.

Weiterbildungen, Vorteile und Anwendungsmöglichkeiten der Erfindung ergeben sich auch aus der nachfolgenden Beschreibung von Ausführungsbeispielen und aus den Figuren.

Die Erfindung wird im Folgenden anhand der Figuren an Ausführungsbeispielen näher erläutert. Es zeigen:
Fig. 1 in Teildarstellung und im Schnitt ein Verschließwerkzeug zum Verschließen von Behältern in Form von Flaschen mit Kronenkorkenverschlüssen;
Fig. 2 in vergrößerter Einzeldarstellung den in einer Ziehringhalterung gehaltenen Ziehring des Verschließwerkzeugs der Figur 1;
Fig. 3 - 11 jeweils in perspektivischer Darstellung den Verschließstempel des Verschließwerkzeugs bei unterschiedlichen Ausführungen des Ziehringhalters bzw. der Zierringaufnahme dieses Halters;
Fig. 12 und 13 jeweils in perspektivischer Einzeldarstellung einen Ziehring zur Verwendung bei dem Verschließwerkzeug der Figur 1;
Fig. 14 eine Darstellung ähnlich Fig. 2 bei einer weiteren Ausführungsform der Erfindung.

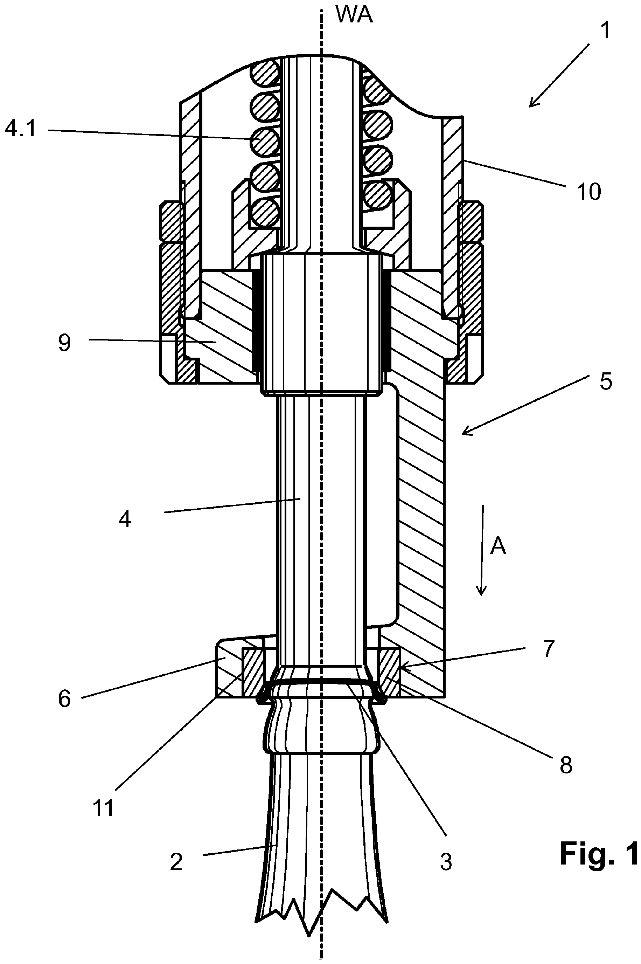

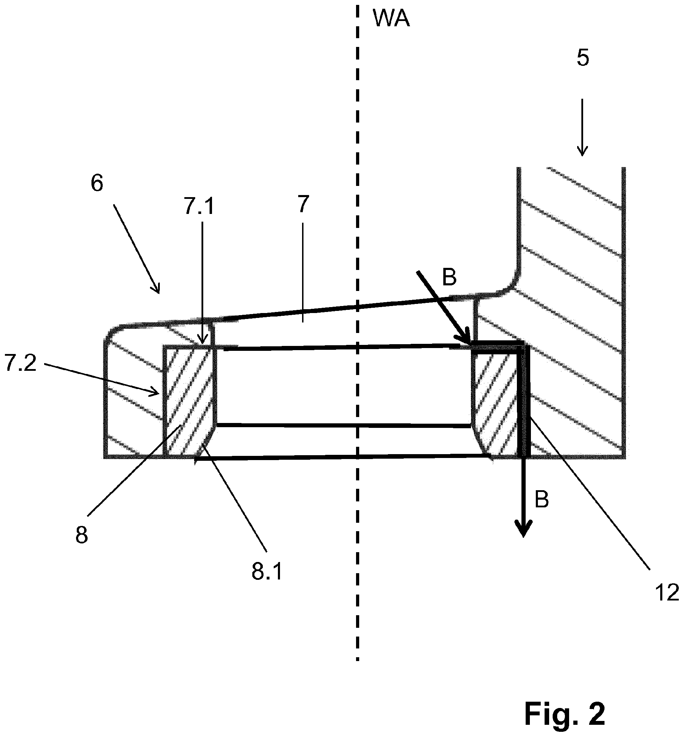

Das in der Figur 1 allgemein mit 1 bezeichnete und in dieser Figur nur teilweise dargestellte Verschließwerkzeug dient zum Verschließen von Behältern in Form von Flaschen 2 mit Kronenkorken 3 und ist der dem Fachmann bekannten Weise Bestandteil einer entsprechenden Verschließmaschine, beispielsweise einer Verschließmaschine umlaufender Bauart, bei der am Umfang eines um eine vertikale Maschinenachse umlaufend antreibbaren Rotors mehrere derartige Verschließwerkzeuge 1 vorgesehen sind. Entsprechend der Darstellung der Figur 1 umfasst das Verschließwerkzeug 1 u.a. einen achsgleich mit einer im Verwendungsfall vertikalen Werkzeugachse WA angeordneten stempelartigen und durch eine Niederhalterfeder 4.1 gefederten Niederhalter 4, mit dem der jeweilige Kronenkorken 3 beim Verschließen gegen die Öffnung der Flasche 2 angepresst und gehalten wird, sowie einen Verschließstempel 5, der an seinem in der Figur 1 unteren Ende einen etwa ringförmigen Ziehringhalter 6 mit einer achsgleich mit der Achse WA angeordneten Öffnung 7 als Aufnahme für einen Ziehring 8 bildet. Der Ziehring 8 ist bei der dargestellten Ausführungsform als kreiszylinderförmiger Ring mit einem abgeschrägten inneren Randbereich 8.1 an der Unterseite ausgebildet (Figur 2). Die Öffnung 7 des Ziehringhalters 6 bildet. für den Ziehring 8 u.a. einen Abstützbereich 7.1 für eine bezogen auf die Achse WA axiale Abstützung, gegen den der Ziehring 8 mit seinem in der Figur 1 oberen Rand anliegt, sowie einen Abstützbereich 7.2 für eine bezogen auf die Achse WA radiale Abstützung, gegen den der Ziehring 8 mit seiner Umfangs- oder Mantelfläche anliegt.

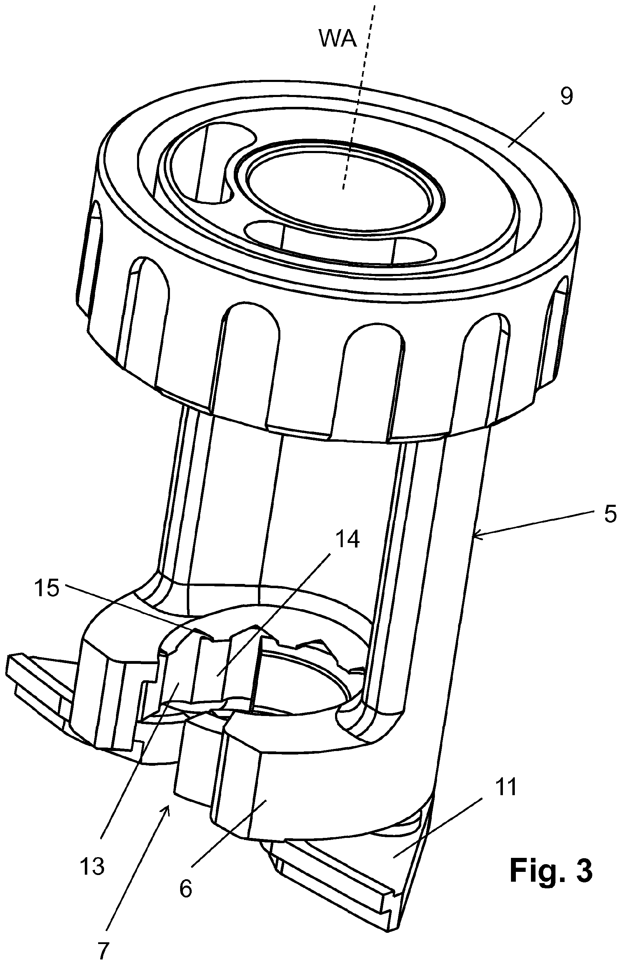

An dem in der Figur 1 oberen Ende ist der Verschließstempel 5 mit einem Verbindungs- oder Halteabschnitt 9 ausgeführt, der den Niederhalter 4 umschließt und mit dem der Verschließstempel 5 austauschbar an einem rohrstückartigen und u.a. den Niederhalter 4 und die Niederhalterfeder 4.1 konzentrisch umschließenden Gehäuseteil 10 befestigt ist. Der Ziehring 8 ist in der Öffnung 7 auf geeignete Weise gehalten, beispielsweise mit einer an der Unterseite des Verschließstempels 5 befestigten Platte 11 (Figur 3). Der Ziehstempel ist beispielsweise zwischen dem Ziehringhalter 6 und dem Verbindungs- oder Halteabschnitt 9 schalenartig, den Niederhalter 4 teilweise umschließend ausgeführt.

Nachstehend werden die dem Verbindungs- oder Halteabschnitt 9 zugewandte Seite des Ziehringhalters 6 als Oberseite und die dem Halte- und Verbindungsabschnitt 9 abgewandte Seite des Ziehringhalters 6 als Unterseite bezeichnet.

Beim Verschließen der betreffenden Flasche 2 wird mit dem Gehäuseteil 10 der Verschließstempel 5 in Richtung der Achse WA nach unten bewegt (Pfeil A) sodass der Rand des auf die Flasche 2 aufgesetzten und dort mit dem Niederhalter 4 angepressten Kronenkorken 3 mit dem Randbereich 8.1 bleibend hinter den Mündungswulst der Flasche 2 verformt und diese somit verschlossen wird. Nach dem Verschließen wird der Verschließstempel wieder angehoben.

Wie ausgeführt, ist zumindest in vorgegebenen oder vorgewählten Zeitabständen eine Behandlung des Verschließwerkzeuges 1, insbesondere auch der mit den Kronenkorkenverschlüssen 3 unmittelbar in Berührung kommenden Abschnitte oder Flächen dieses Werkzeugs, d.h. insbesondere des Ziehringhalters 6 und des Ziehringes 8 mit dem flüssigen und/oder gas- und/oder dampfförmigen Reinigungs- und/oder Desinfektionsmedium erforderlich. Entsprechend der der Erfindung zugrunde liegenden Erkenntnis muss diese Behandlung nicht nur an den unmittelbar freiliegenden Flächen des Verschließwerkzeugs 1 und dabei des Ziehringhalters 6 und des Ziehringes 8 erfolgen, sondern dass ein besonders kritischer und daher ebenfalls zu behandelnder Bereich auch der Übergang oder Spalt 12 zwischen der Außenfläche des Ziehringes 8 und dem diesen Ziehring 8 umschließenden Ziehringhalter 6 ist.

Um eine intensive Behandlung des Spaltes 12 zu ermöglichen, schlägt daher die Erfindung abweichend vom Stand der Technik vor, die die Ziehringaufnahme bildende Öffnung 7 zumindest an den Abstützungen 7.1 und 7.2 und/oder den Ziehring 8 an seiner Umfangsfläche und beispielsweise auch an seiner Stirnfläche zu profilieren, und zwar zur Ausbildung einer Vielzahl von erhabenen Bereichen und an diese angrenzenden, beispielsweise diese auch umschließende Vertiefungen oder Gräben. Dort, wo die Öffnung 7 und/oder der Ziehring 8 diese Profilierung aufweisen, erfolgt die Anlage des Ziehringes 8 am Ziehringhalter 6 dann nur an den von den erhabenen Bereichen gebildeten Anlageflächen, deren Flächenmaß in Summe deutlich kleiner ist als dasjenige Flächenmaß, welches die Anlageflächen zwischen dem Ziehringhalter 6 und dem Ziehring 8 ohne diese Profilierung in Summe aufweisen würden. Die Profilierung ist aber insbesondere auch so ausgeführt, dass sich im Spalt 12 entlang der Vielzahl der erhabenen Bereiche und diese ggs. auch umschließend ein von dem flüssigen und/oder gas- und/oder dampfförmigen Behandlungsmedium durchströmbarer Strömungsweg ergibt, vorzugsweise zwischen der Ober- und Unterseite des Ziehringes 8, wie dies in der Figur 2 mit den Pfeilen B angedeutet ist.

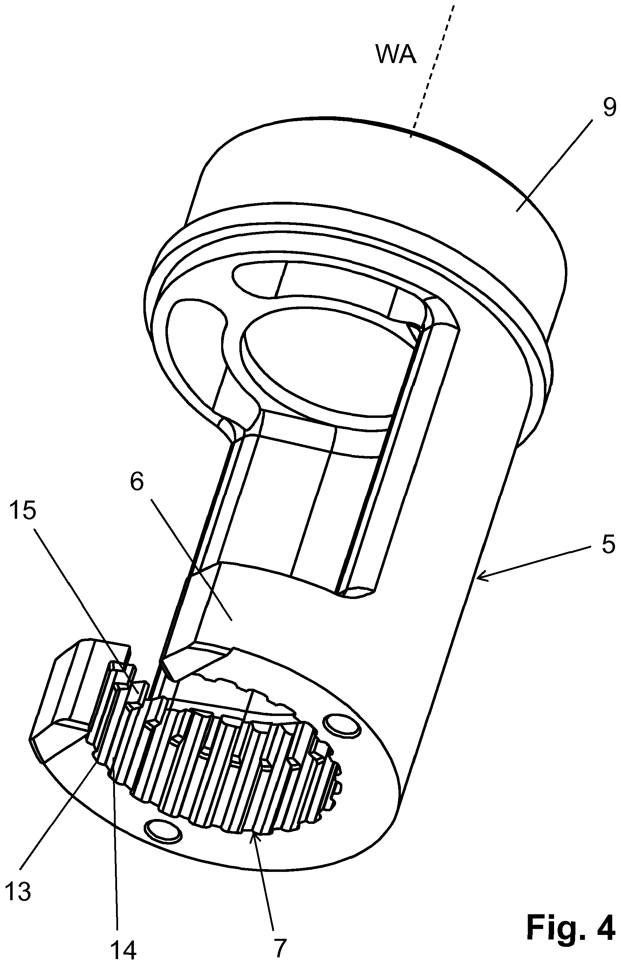

Wie die Figuren 3 - 11 zeigen, kann die Profilierung an den Abstützbereichen 7.1 und 7.2 des Ziehringhalters 6 auf unterschiedlichste Weise realisiert sein, beispielsweise bei dem Ziehstempel 5 der Figuren 3 und 4 dadurch, dass die Öffnung 7 an ihrer den radialen Abstützbereich bildenden Innenfläche mit einer Vielzahl von nutenförmigen Vertiefungen 13 versehen ist, die sich jeweils parallel zur Achse WA erstrecken und an der Oberseite und an der Unterseite des Ziehringhalters 6 offen sind. Die jeweils zwischen zwei Nuten 13 gebildeten Stege 14, die mit ihrer Längserstreckung ebenfalls parallel zur Achse WA angeordnet sind, weisen im Bereich ihres oberen Endes, d.h. im Bereich der Oberseite des Ziehringhalters 6 jeweils einen bezogen auf die Achse WA radial nach innen in die Öffnung 7 vorstehenden Vorsprung 15 auf. Die Vorsprünge 15 bilden in Summe den axialen Abstützbereich und die Stege 14 in Summe den radialen Abstützbereich der Öffnung 7. Über die Nuten 13 ist eine Strömung des Behandlungsmediums durch den Spalt 12 möglich. Die in den Figuren 3 und 4 dargestellten Ausführungsformen unterscheiden sich voneinander lediglich dadurch, dass die axialen und radialen Abstützbereiche der Öffnung 7 bei der Ausführung der Figur 4 feiner strukturiert sind als bei der Ausführung der Figur 3, d.h. die Anzahl der Nuten 13, Stege 14 und Vorsprünge 15 bei dem Verschließstempel 5 der Figur 4 größer ist als dem Verschließstempel 5 der Figur 3.

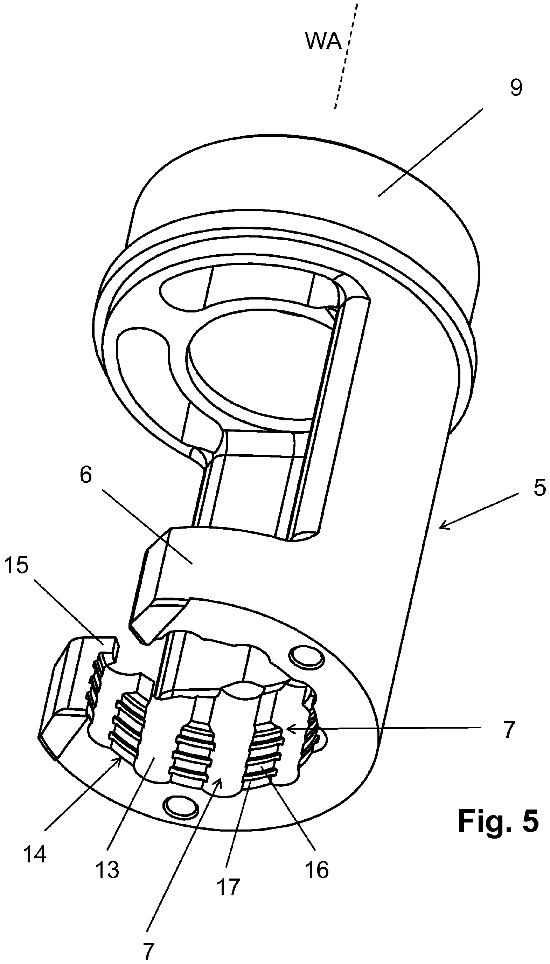

Die Figur 5 zeigt als weitere Ausführungsform einen Verschließstempel 5, bei dem die Abstützbereiche 7.1 und 7.2 der Öffnung 7 ähnlich den Figuren 3 und 4 strukturiert sind, und zwar mit Nuten 13 und Stegen 14, welch letztere jeweils an der Oberseite des Ziehringhalters 6 mit einem Vorsprung 15 versehen sind. Abweichend von den Figuren 3 und 4 sind bei dem Verschließstempel 5 der Figur 5 aber die Stege 14 ihrerseits zusätzlich profiliert, und zwar wiederum zur Ausbildung erhabener Bereiche, die die Anlageflächen für den Ziehring 8 bilden, sowie unter Ausbildung von an die erhabenen Bereiche angrenzenden Vertiefungen oder Gräben. Bei der dargestellten Ausführungsform sind die Stege 14 hierfür an ihrer der Achse WA zugewandten Fläche mit zusätzlichen, beidendig in jeweils eine Nut 13 mündenden Nuten 16 und mit zwischen diesen gebildeten Stegen 17 versehen, die in Summe den radialen Anlagebereich für den Ziehring 8 bilden und die sich beispielsweise ebenso wie die Nuten 16 tangential zu einem gedachten, die Achse WA umschließenden Kreis erstrecken. Durch die zusätzlichen Nuten 16 und Stege 17 ist eine sich mehrfach verzweigende Strömung des Behandlungsmediums im Spalt 12 möglich, d.h. eine Strömung, die sowohl eine bezogen auf die Achse WA axiale, radiale und auch tangentiale Komponente aufweist.

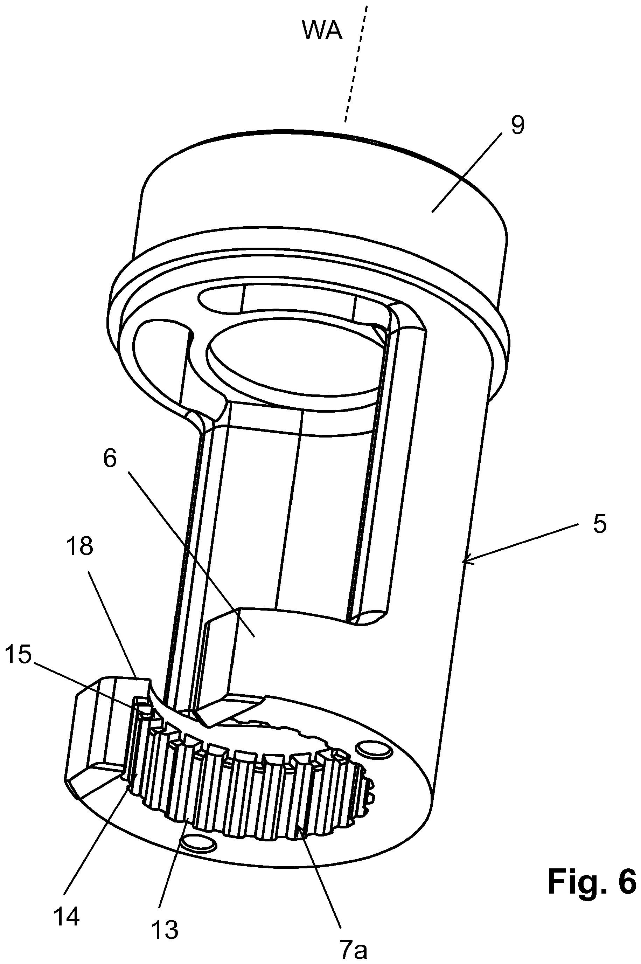

Während bei den Verschließstempeln 4 der Figuren 3 - 5 die Nuten 13 und die Vorsprünge 15 bis an die den Halteabschnitt 9 zugewandte Oberseite des Ziehringhalters 6 reichen, zeigen die Figuren 6 - 11 Ausführungsformen des Verschließstempels 5, bei denen die die Ziehringaufnahme bildende Öffnung 7 an der Oberseite des Ziehringhalters 6 insbesondere auch zur Erhöhung der mechanischen Festigkeit auf einen Durchmesser verengt ist, der kleiner ist als der Außendurchmesser des Ziehringes 8, d.h. die in diesen Figuren allgemein mit 7a bezeichnete und der Öffnung 7 entsprechende Öffnung weist an der Oberseite des Ziehringhalters 6 einen die Achse WA umschließenden durchgehenden und in die Öffnung 7a hineinragenden ringförmigen Absatz oder Vorsprung 18 auf. Abgesehen hiervon ist der Verschließstempel 5 der Figur 6 an den Abstützbereichen für den Ziehring 8 ähnlich dem Verschlussstempel 5 der Figur 4 profiliert, d.h. mit den Nuten 13 und Stegen 14 versehen, die an ihrem oberen Ende die Vorsprünge 15 aufweisen. Die Nuten 13 reichen von der Unterseite des Ziehringhalters 6 bzw. der Öffnung 7 bis an den Vorsprung 18. Die Vorsprünge 15 schließen ausgehend von der Oberseite des Ziehringhalters 6 an den Vorsprung 18 an und sind entlang des Vorsprungs 18 voneinander beabstandet, sodass sich im Spalt 12 zwischen dem Ziehring 8 und dem Ziehringhalter 6 wiederum eine Vielzahl von Strömungskanälen ergibt, die sich zwischen der Oberseite und der Unterseite des Ziehringhalters erstrecken und von den Nuten 16 und den Zwischenräumen zwischen den Vorsprüngen 15 gebildet sind.

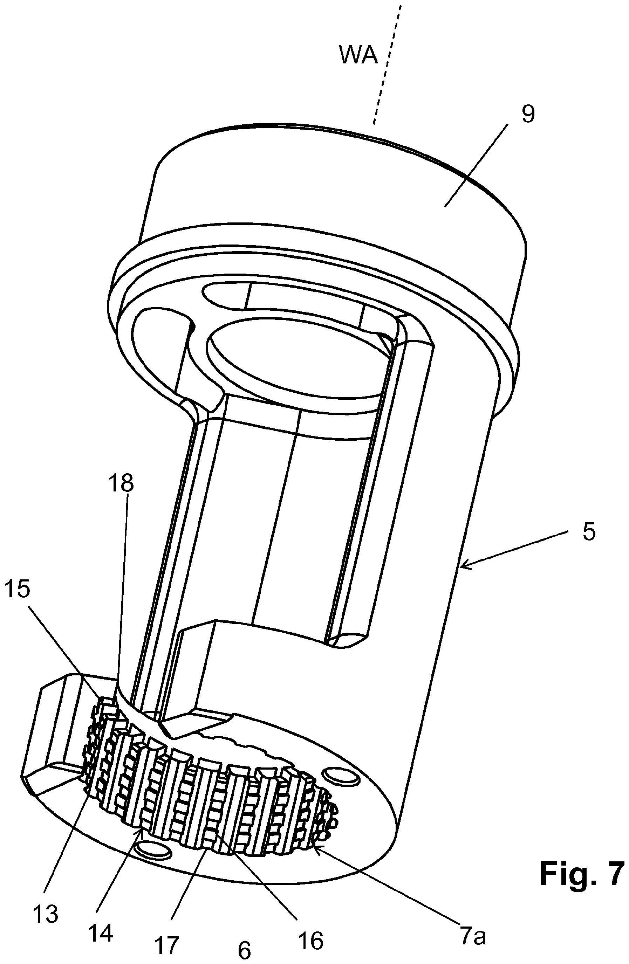

Die Figur 7 zeigt als weitere Ausführungsform einen Verschließstempel 5, der sich von dem Verschließstempel 4 der Figur 6 dadurch unterscheidet, dass die Stege 14 zusätzlich profiliert sind, d.h. die zusätzlichen Nuten 16 und Stege 17 aufweisen, die bei dieser Ausführungsform wegen ihrer kurzen Länge auch als Vorsprünge und Vertiefungen erscheinen. Durch die zusätzlichen Nuten 16 und Stege 17 ist wiederum eine sich mehrfach verzweigende Strömung des Behandlungsmediums im Spalt 12 möglich, d.h. eine Strömung, die sowohl eine bezogen auf die Achse WA axiale, radiale und auch tangentiale Komponente aufweist.

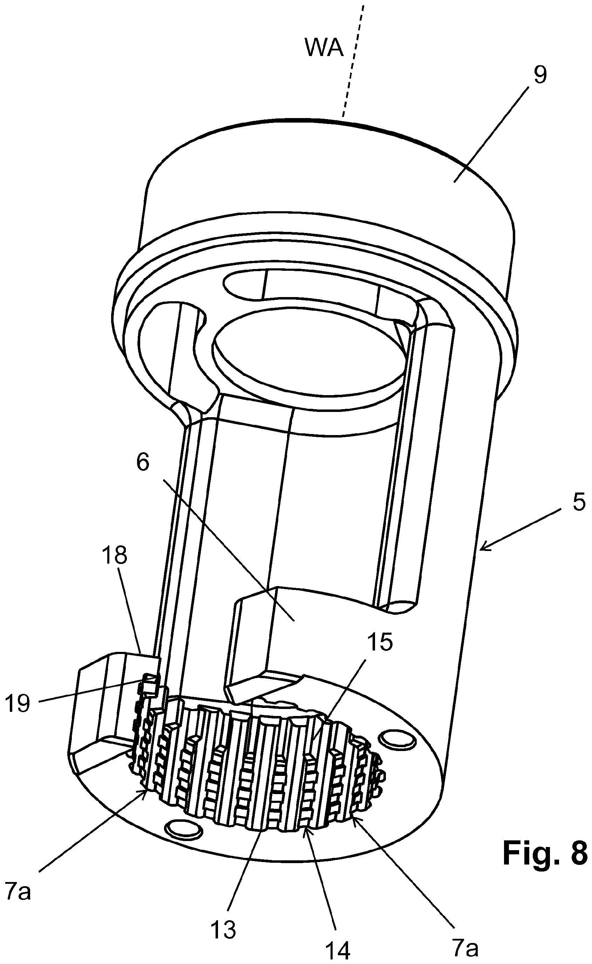

Die Figur 8 zeigt als weitere Ausführungsform einen Verschließstempel 5, der sich von dem Verschließstempel 5 der Figur 7 im Wesentlichen nur dadurch unterscheidet, dass die Vorsprünge 15 jeweils von dem Vorsprung 18 bezogen auf die Achse WA axial beabstandet sind, sodass sich zwischen dem Vorsprung 18 und den Vorsprüngen 15 ein ringförmiger Strömungskanal 19 ergibt, in den die Nuten 16 hineinreichen und der bezogen auf die Achse WA innenliegend offen ist.

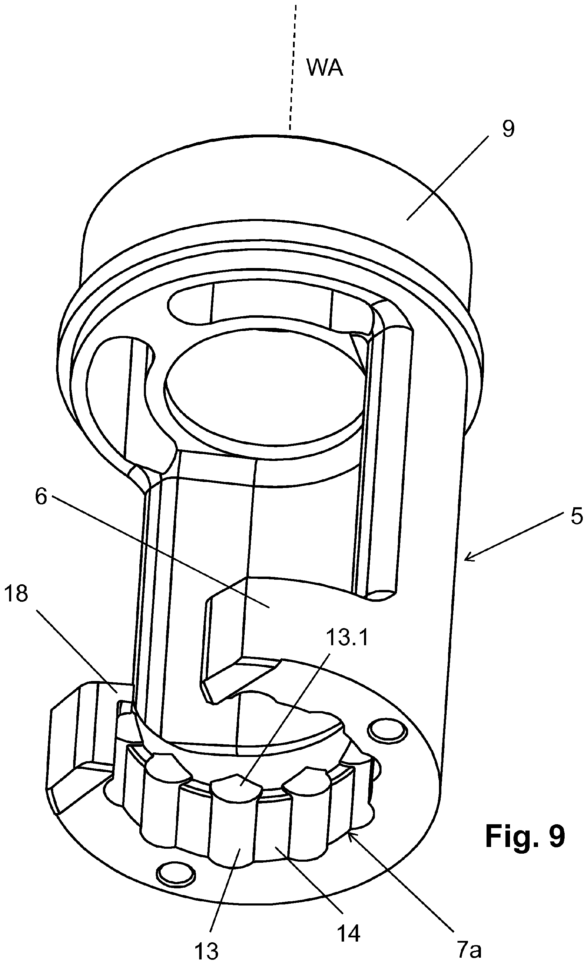

Die Figur 9 zeigt als weitere Ausführungsform einen Verschließstempel 5, bei dem der Vorsprung 18 den axialen Abstützbereich der Öffnung 7a für den Ziehring 8 bildet und die an der Unterseite des Ziehringhalters 6 offenen Nuten 16 bis in den Vorsprung 18 reichen und dort an einer der Achse WA zugewandten Öffnung 13.1 offen sind.

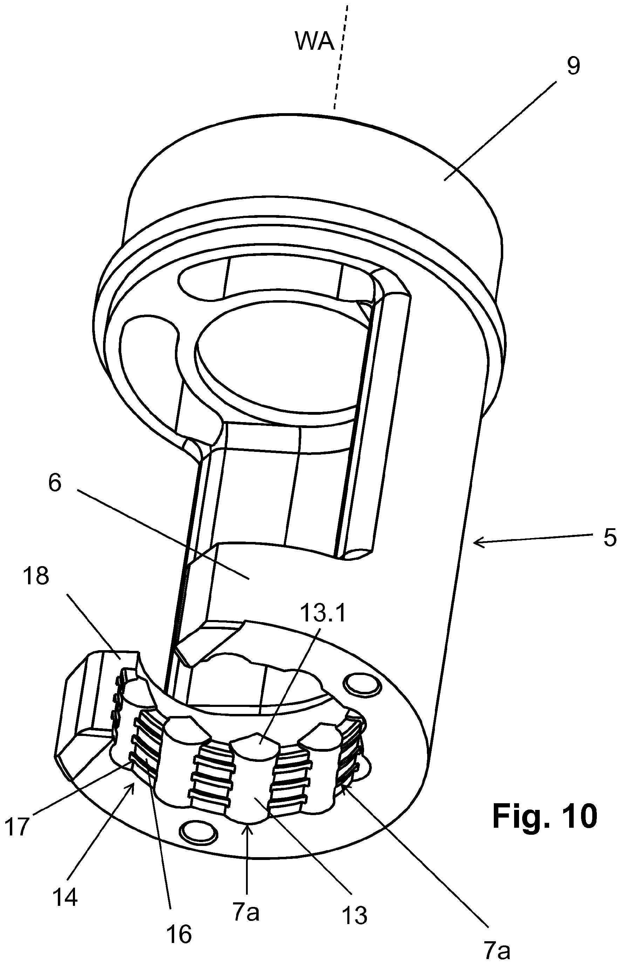

Die Figur 10 zeigt eine Ausführung des Verschließstempels 5, die sich von der Ausführung der Figur 9 dadurch unterscheidet, dass die Stege 14 wiederum die zusätzlichen, beidendig in jeweils eine Nut 13 mündenden Nuten 16 und die Stege 17 aufweisen.

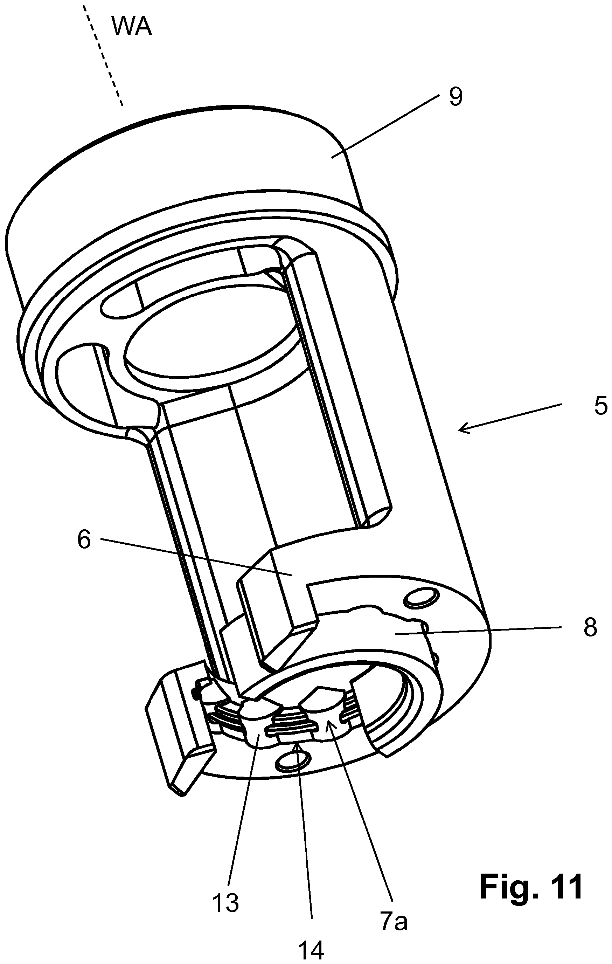

Die Figur 11 zeigt als weitere Ausführung einen Verschließstempel 5, dessen die Ziehringaufnahme bildende Öffnung 7a an den Abstützbereichen für den Ziehring 8 ähnlich ausgebildet ist wie bei dem Verschließstempel 5 der Figur 10, wobei allerdings der Ziehringhalter 6 in Richtung der Achse WA eine reduzierte Höhe aufweist, sodass der in der Figur 11 auch teilweise dargestellte Ziehring 8 über die Unterseite des Ziehringhalters 6 vorsteht.

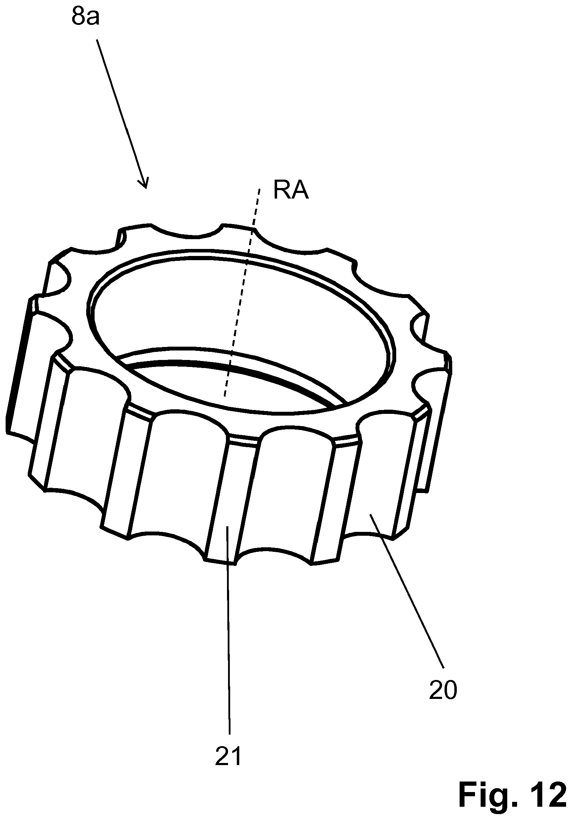

Vorstehend wurde beschrieben, dass zur Erzeugung von Strömungskanälen im Spalt 12 zwischen dem Ziehringhalter 6 und dem Ziehring 8 der Ziehringhalter 6 an den Anlagebereichen 7.1 und 7.2 profiliert ist. Anstelle hiervon oder aber zusätzlich hierzu kann auch der Ziehring 8 an seinem Umfangsbereich und/oder an seiner Stirnfläche 8.1 profiliert sein. Die Figur 12 zeigt daher als weitere Ausführungsform einen Ziehring 8a, der an seinem Umfang mit einer Vielzahl von Nuten 20 und zwischenliegenden Stegen 21 ausgebildet ist. Die Nuten 20 sind zum Umfang sowie an der Ober- und Unterseite des Ziehringes 8a offen. Die Stege 21 bilden Anlagebereiche oder -flächen, mit dem sich der Ziehring 8a in der die Ziehringaufnahme bildenden Öffnung 7 oder 7a radial abstützt. Die Nuten 20 und Stege 21 sind mit ihrer Längserstreckung parallel oder im Wesentlichen parallel zu der Ringachse RA des Ziehrings 8a orientiert.

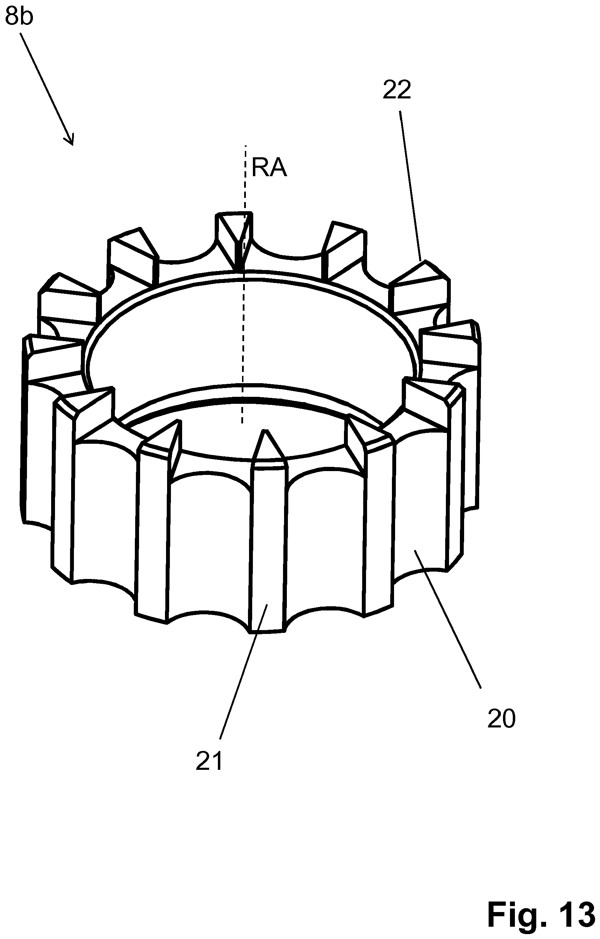

Die Figur 13 zeigt als weitere Ausführungsform einen Ziehring 8b, der sich von dem Ziehring 8a dadurch unterscheidet, dass sich jeder Stege 21 an der Oberseite des Ziehringes in einem Vorsprung 22 fortsetzt und dort jede Nut 20 in den Zwischenraum zwischen zwei Vorsprüngen 22 mündet, die in ihrer Gesamtheit dann die axiale Anlage für den Ziehring 8b in der die Ziehringaufnahme bildenden Öffnung 7 bzw. 7a bilden.

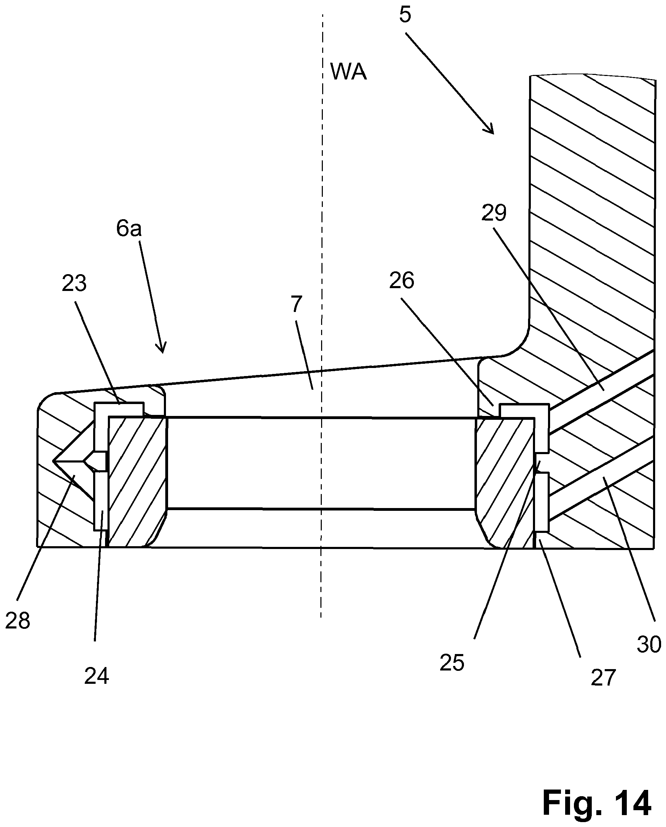

Die Fig. 14 zeigt in vergrößerter Einzeldarstellung den am unteren Ende des Verschließstempels 5 ausgebildeten Ziehringhalter 6a einer weiteren Ausführungsform des erfindungsgemäßen Verschließwerkzeugs. Der Ziehringhalter 6a ist wiederum mit der achsgleich mit der Achse WA angeordneten und ausgebildeten Öffnung 7 versehen, die die Aufnahme für den Ziehring 8 bildet und die wiederum zur Ausbildung einer zur axialen Abstützung des Ziehrings 8 dienen Stufe in ihrem oberen Bereich bzw. an der Oberseite des Ziehringhalters 6a einen reduzierten Querschnitt aufweist.

In der Öffnung 7 sind zwei, die Werkzeugachse WA ringförmig umschließende Nuten 23 und 24 ausgebildet, die jeweils zur Öffnung 7 hin offen sind und von denen Nut 23 an dem zur axialen Abstützung des Ziehringes 8 dienenden Stufe und an der den Ziehring 8 umschließenden Innenfläche der Öffnung 7 ausgebildet ist, während die Nut 23 ausschließlich an der den Ziehring umschließenden Innenfläche der Öffnung 7 ausgeführt ist. Die beiden Nuten 23 und 24 sind bezogen auf die Werkzeugachse WA axial gegeneinander versetzt und zumindest entlang des größeren Teils des Umfangs des Ziehringes 8 durch einen die Werkzeugachse WA umschließenden ringförmigen Steg 25 getrennt. An den dem Steg 25 entfernt liegenden Rändern sind die Nuten 23 und 24 durch jeweils einen ringförmigen, die Werkzeugachse WA ringförmig umschließenden weiteren Steg 26 (Nut 23) bzw. 27 (Nut 24) begrenzt. Dabei bilden der Steg 26 den axialen Abstützbereich und die Stege 25 und 27 die radiale Abstützbereiche für den Ziehring 8. Über eine Aussparung 28 an der Innenfläche der Öffnung 7 können die beiden Nuten 23 und 24 vorteilhafter Weise strömungsmäßig miteinander verbunden sein, wodurch die Spülung noch weiter verbessert wird. Weiterhin sind im Ziehringhalter 6 zwei Kanäle 29 und 30 vorgesehen, von denen der Kanal 29 in die Nut 23 und der Kanal 30 in die Nut 24 mündet und die an einer Außenfläche des Ziehringhalters 6a offen sind. Die beiden Kanäle 29 und 23 sind dabei bevorzugt bezogen auf die Werkzeugachse WA gegenüber der Ausnehmung 28 um 180° versetzt vorgesehen und ermöglichen ein Zuführen sowie Abführen des flüssigen und/oder dampfförmigen und/oder gasförmigen Behandlungsmediums (insbesondere Reinigungs- und/oder Sterilisationsmediums) zum Durchströmen der Nuten 23 und 24 und dabei auch des zwischen dem Ziehringhalter 6a und dem Ziehring 8 gebildeten Spalts, wobei das Behandlungsmedium auch in den Spalt zwischen dem Ziehring 8 und den Stegen 25, 26 und 27 gelangt.

Die Erfindung wurde voranstehend an Ausführungsbeispielen beschrieben. Es versteht sich, dass zahlreiche Änderungen sowie Abwandlungen möglich sind. So wurde vorstehend ausgeführt, dass sich die Nuten 13 und 20 parallel zur Achse WA oder RA erstrecken. Auch eine andere Orientierung dieser Nuten ist möglich, beispielsweise schräg oder schraubenförmig zur Achse WA oder RA. Auch ist eine gradlinige Ausbildung der Nuten nicht unbedingt erforderlich. Weiterhin ist es auch möglich, den Ziehring 8, 8a oder 8b schwimmend, d.h. mit einem gewissen axialen und/oder radialen Spiel in der Öffnung 7 bzw. 7a des Ziehringhalters 6 anzuordnen.

### Bezugszeichenliste

- 1: Verschließwerkzeug
- 2: Flasche
- 3: Kronenkorken
- 4: Niederhalter
- 4.1: Niederhalterfeder
- 5: Verschließstempel
- 6, 6a: Ziehringhalter
- 7, 7a: Öffnung
- 7.1, 7.2: Abstützbereich
- 8, 8a, 8b: Ziehring
- 8.1: Ziehringrand
- 9: Verbindungs- oder Halteabschnitt
- 10: Gehäuseteil
- 11: Platte
- 12: Spalt
- 13: Nut
- 13.1: Öffnung
- 14: Steg
- 15: Vorsprung
- 16: Nut
- 17: Steg
- 18: Vorsprung
- 19: Strömungskanal
- 20: Nut
- 21: Steg
- 22: Vorsprung
- 23, 24: Nut
- 25, 26, 27: Steg
- 28: Ausnehmung
- 29, 30: Kanal
- A: Bewegung des Verschließstempels 5
- B: Strömung durch den Spalt 12
- RA: Ringachse
- WA: Achse des Verschließwerkzeugs 1

## Patentansprüche

1. Verschließwerkzeug zum Verschließen von Flaschen (2) mit Kronenkorken (3) oder dergleichen Verschlüssen mittels eines aus einer Ausgangsposition absenkbaren sowie in diese Ausgangsposition rückführbaren und in einer Ziehringaufnahme oder Öffnung (7, 7a) eines Ziehringhalters (6, 6a) aufgenommenen Ziehringes (8, 8a, 8b), der sich an in der Ziehringaufnahme oder Öffnung (7, 7a) gebildeten Abstützbereichen (7.1, 7.2) bezogen auf die Werkzeugachse (WA) radial sowie axial abstützt,
**dadurch gekennzeichnet,**
**dass** im Spalt (12) zwischen der Ziehringaufnahme oder Öffnung (7, 7a) und benachbarten Flächen des Ziehringes (8, 8a, 8b) wenigstens ein Strömungsweg gebildet ist, der von einem flüssigen und/oder dampfförmigen und/oder gasförmigen Behandlungsmedium durchströmbar ist.

2. Verschließwerkzeug nach Anspruch 1, **dadurch gekennzeichnet, dass** im Spalt (12) zwischen der Ziehringaufnahme oder Öffnung (7, 7a) und benachbarten Flächen des Ziehringes (8, 8a, 8b) eine Vielzahl von Strömungswegen gebildet sind, die von einem flüssigen und/oder dampfförmigen und/oder gasförmigen Behandlungsmedium durchströmbar sind.

3. Verschließwerkzeug nach Anspruch 1 oder 2, **dadurch gekennzeichnet, dass** der wenigstens eine Strömungsweg oder die Strömungswege jeweils durchgehend zwischen einer Unterseite und einer Oberseite des Ziehringhalters (6, 6a) und des Ziehringes (8, 8a, 8b) ausgebildet sind und sich beispielsweise bezogen auf die Werkzeugachse (WA) zumindest axial oder im Wesentlichen axial, bevorzugt auch zusätzlich radial oder im Wesentlichen radial und/oder tangential oder im Wesentlichen tangential erstrecken.

4. Verschließwerkzeug nach Anspruch 2 oder 3, **dadurch gekennzeichnet, dass** die die Ziehringaufnahme oder die diese bildende Öffnung (7, 7a) an ihrer Innenfläche und/oder der Ziehring (8, 8a, 8b) an der sich am Ziehringhalter (6, 6a) abstützenden Umfangs- und/oder Stirnfläche profiliert sind, und zwar jeweils zur Ausbildung einer Vielzahl von erhabenen Bereichen und angrenzenden, diese erhabenen Bereiche beispielsweise auch zumindest teilweise umschließenden Vertiefungen oder Gräben, und dass die erhabenen Bereiche in Summe die Anlagefläche der Ziehringaufnahme und/oder die Anlagefläche des Ziehrings (8, 8a, 8b) bilden.

5. Verschließwerkzeug nach Anspruch 4, **dadurch gekennzeichnet, dass** das Flächenmaß der Summe der von den erhabenen Bereichen gebildeten Anlage- oder Abstützflächen für den Ziehring (8, 8a, 8b) deutlich kleiner ist als das Flächenmaß, das nicht profilierte Anlage- oder Abstützflächen für den Ziehring (8, 8a, 8b) in Summe aufweisen würden, beispielsweise um wenigstens 50%.

6. Verschließwerkzeug nach Anspruch 4 oder 5, **dadurch gekennzeichnet, dass** die Profilierung in Form von Nuten (13, 16) und dazwischen liegenden, die Anlage- oder Abstützflächen bildenden Stegen (14, 17, 21) ausgeführt ist.

7. Verschließwerkzeug nach einem der Ansprüche 4 - 6, **dadurch gekennzeichnet, dass** die Profilierung für eine bezogen auf die Werkzeugachse (WA) axiale Abstützung des Ziehringes (8, 8a, 8b) von einer Vielzahl von Vorsprüngen (15, 22) gebildet ist, die voneinander beabstandet und um die Werkzeugachse (WA) oder die Achse (RA) des Ziehringes (8b) verteilt in der Ziehringaufnahme oder Öffnung (7, 7a) und/oder an einer Stirnfläche des Ziehringes (8b) vorgesehen sind.

8. Verschließwerkzeug nach einem der Ansprüche 6 oder 7, **dadurch gekennzeichnet, dass** die die Profilierung bildenden Nuten (13, 20) mit ihrer Längserstreckung parallel oder im Wesentlichen parallel zur Werkzeugachse (WA) oder zu einer Achse (RA) des Ziehringes (8a, 8b) oder schräg oder schraubenförmig in Bezug auf diese Achsen (WA, RA) ausgeführt sind.

9. Verschließwerkzeug nach einem der Ansprüche 6 - 8, **dadurch gekennzeichnet, dass** in den Stegen (14) Vertiefungen oder zusätzliche Nuten (16) vorgesehen sind, die jeweils beidendig in eine zwei Stege (14) beabstandende Nut (13) münden und beispielsweise bezogen auf die Maschinenachse (WA) oder bezogen auf die Achse (RA) des Ziehringes (8, 8a, 8b) tangential oder im Wesentlichen tangential orientiert sind.

10. Verschließwerkzeug nach einem der Ansprüche 6 - 9, **dadurch gekennzeichnet, dass** die zur axialen Abstützung des Ziehringes (8, 8a, 8b) dienenden Vorsprünge (15) an jeweils einem Steg (14) ausgebildet sind und vorzugsweise in einen gemeinsamen Vorsprung (18) übergehen, der ringförmig ausgebildet und die Werkzeugachse (WA) umschließend in die Ziehringaufnahme oder in die diese Aufnahme bildende Öffnung (7a) hineinreicht.

11. Verschließwerkzeug nach einem der vorhergehenden Ansprüche, **dadurch gekennzeichnet, dass** im Ziehringhalter (6a) wenigstens ein in den Spalt zwischen dem Ziehringhalter (6a) und benachbarten Flächen des Ziehringes (8, 8a, 8b) mündender Kanal (29, 30) zum Zuführen und/oder Abführen des Behandlungsmediums vorgesehen ist, wobei der wenigstens eine Kanal (29, 30) vorzugsweise an einer Fläche des Ziehringhalters (6a) außerhalb der Ziehringaufnahme oder der diese Aufnahme bildenden Öffnung (7, 7a) offen ist.

12. Verschließwerkzeug nach Anspruch 11, **dadurch gekennzeichnet, dass** der wenigstens eine Kanal (29, 30) in wenigstens eine in der Ziehringaufnahme oder Öffnung (7) ausgebildete, die Werkzeugachse (WA) beispielsweise zumindest teilweise umschließende Nut (23, 24) mündet, wobei beispielsweise zwei in Bezug auf die Werkzeugachse (WA) axial gegeneinander versetzte Nuten (23, 24) vorgesehen sind, in die jeweils wenigstens ein Kanal (29, 30) mündet und die vorzugsweise strömungsmäßig miteinander verbunden sind.

13. Verschließwerkzeug nach Anspruch 12, **dadurch gekennzeichnet, dass** die wenigstens eine Nut (23, 24) seitlich begrenzende Stege (25, 26, 27) die axialen sowie radialen Abstützbereiche des Ziehringes bilden, und dass dieser Abstützbereich bevorzugt die Profilierung aufweist.

14. Ziehring zur Verwendung bei einem Verschließwerkzeug nach einem der vorhergehenden Ansprüche, **dadurch gekennzeichnet, dass** der Verschließring (8a, 8b) an seiner Umfangs- oder Mantelfläche und/oder an wenigstens einer Stirnfläche die Profilierung aufweist.

## Claims

1. Closing tool for closing bottles (2) with crown corks (3) or closures of this type by means of a drawing ring (8, 8a, 8b), which can be lowered from a starting position and returned into this starting position and can be accommodated in a drawing ring receptacle or opening (7, 7a) of a drawing ring holder (6, 6a), this drawing ring supporting itself on supporting regions (7.1, 7.2) formed in drawing ring receptacle or opening (7, 7a), in a radial and axial manner relative to the tool axis (WA),
**characterised in that**
at least one flow path is formed in the gap (12) between the drawing ring receptacle or opening (7, 7a) and adjacent surfaces of the drawing ring (8, 8a, 8b), through which a liquid and/or vaporous and/or gaseous treatment medium can flow.

2. Closing tool according to claim 1, **characterised in that** a plurality of flow paths are formed in the gap (12) between the drawing ring receptacle or opening (7, 7a) and adjacent surfaces of the drawing ring (8, 8a, 8b), through which a liquid and/or vaporous and/or gaseous treatment medium can flow.

3. Closing tool according to claim 1 or 2, **characterised in that** the at least one flow path or the flow paths are formed in each case passing continuously between an under side and an upper side of the drawing ring holder (6, 6a) and the drawing ring (8, 8a, 8b), and, related for example to the tool axis (WA), extend at least axially or essentially axially, and preferably also additionally radially or essentially radially and/or tangentially or essentially tangentially.

4. Closing tool according to claim 2 or 3, **characterised in that** the drawing ring receptacle or the opening forming this (7, 7a) is profiled on its inner surface, and/or the drawing ring (8, 8a, 8b) is profiled on the circumferential surface and/or face surface supporting it at the drawing ring holder (6, 6a), and specifically in each case for the formation of a plurality of elevated regions and adjacent depressions or troughs, for example at least partially surrounding these elevated regions, and that the elevated regions taken as a whole sum total form the contact surface of the drawing ring receptacle and/or the contact surface of the drawing ring (8, 8a, 8b).

5. Closing tool according to claim 4, **characterised in that** the surface dimension of the whole sum total of the contact or support surfaces for the drawing ring (8, 8a, 8b), formed by the elevated regions, is perceptibly smaller than the surface dimension which non-profiled contact or support surfaces for the drawing ring (8, 8a, 8b) would exhibit as a whole sum total, for example by at least 50%.

6. Closing tool according to claim 4 or 5, **characterised in that** the profiling is configured in the form of grooves (13, 16) and, located between them, webs (14, 17, 21) forming the contact or support surfaces.

7. Closing tool according to any one of claims 4-6, **characterised in that** the profiling for a support of the drawing ring (8, 8a, 8b), axial in relation to the tool axis (WA), is formed by a plurality of projections (15, 22), which are spaced apart from one another and are provided distributed about the tool axis (WA) or the axis (RA) of the drawing ring (8b), in the drawing ring receptacle or opening (7, 7a) and/or on a face surface of the drawing ring (8b).

8. Closing tool according to any one of claims 6 or 7, **characterised in that** the grooves (13, 20) forming the profiling are configured with their longitudinal extension parallel or essentially parallel to the tool axis (WA) or to an axis (RA) of the drawing ring (8a, 8b) or obliquely or in helical fashion in relation to these axes (WA, RA).

9. Closing tool according to any one of claims 6-8, **characterised in that** depressions or additional grooves (16) are provided in the webs (14), which in each case open on both sides into a groove (13) forming a spacing between two webs (14), and, related for example to the machine axis (WA) or related to the axis (RA) of the drawing ring (8, 8a, 8b), are oriented tangentially or essentially tangentially.

10. Closing tool according to any one of claims 6-9, **characterised in that** the projections (15) serving to provide the axial support of the drawing ring (8, 8a, 8b) are formed in each case at a web (14) and preferably merge into a common projection (18), which is configured in ring form and which, surrounding the tool axis (WA), extends into the drawing ring receptacle or into the opening (7a) forming this receptacle.

11. Closing tool according to any one of the preceding claims, **characterised in that** in the drawing ring holder (6a) at least one channel (29, 30) is provided, opening into the gap between the drawing ring holder (6a) and adjacent surfaces of the drawing ring (8, 8a, 8b), for delivering and/or removing the treatment medium, wherein the at least one channel (29, 30) is preferably open at a surface of the drawing ring holder (6a) outside the drawing ring receptacle or the opening (7, 7a) forming this receptacle.

12. Closing tool according to claim 11, **characterised in that** the at least one channel (29, 30) opens into at least one groove (23, 24), formed in the drawing ring receptacle or opening (7), and, for example, surrounding the tool axis (WA) at least partially, wherein, for example, two grooves (23, 24) are provided, offset to one another axially in relation to the tool axis (WA), into which in each case at least one channel (29, 30) opens, and which are preferably connected to one another such as to allow a flow.

13. Closing tool according to claim 12, **characterised in that** the at least one groove (23, 24) comprises laterally delimited webs (25, 26, 27), which form axial and radial supporting regions of the drawing ring, and that this supporting region preferably comprises the profiling.

14. Drawing ring for use with a closing tool according to any one of the preceding claims, **characterised in that** the drawing ring (8a, 8b) comprises the profiling at its circumferential or casing surface and/or on at least one face surface.

## Revendications

1. Outil de fermeture servant à fermer des bouteilles (2) avec des bouchons-couronnes (3) ou des systèmes de fermeture similaires au moyen d'une bague d'enfoncement (8, 8a, 8b) pouvant être abaissée depuis une position de départ ainsi que pouvant être ramenée dans ladite position de départ et logée dans un logement de bague d'enfoncement ou dans une ouverture (7, 7a) d'un porte-bague d'enfoncement (6, 6a), qui prend appui de manière radiale ainsi que de manière axiale au niveau de zones d'appui (7.1, 7.2) formées dans le logement de bague d'enfoncement ou dans l'ouverture (7, 7a) par rapport à l'axe d'outil (WA),
**caractérisé en ce**
**qu'**est formé, dans la fente (12) entre le logement de bague d'enfoncement ou l'ouverture (7, 7a) et des faces adjacentes de la bague d'enfoncement (8, 8a, 8b), au moins un trajet d'écoulement, qui peut être traversé par un milieu de traitement liquide et/ou sous forme de vapeur et/ou sous forme de gaz.

2. Outil de fermeture selon la revendication 1, **caractérisé en ce que** sont formés, dans la fente (12) entre le logement de bague d'enfoncement ou l'ouverture (7, 7a) et des faces adjacentes de la bague d'enfoncement (8, 8a, 8b), une pluralité de trajets d'écoulement, qui peuvent être traversés par un milieu de traitement liquide et/ou sous forme de vapeur et/ou sous forme de gaz.

3. Outil de fermeture selon la revendication 1 ou 2, **caractérisé en ce que** l'au moins un trajet d'écoulement ou les trajets d'écoulement sont réalisés respectivement de manière traversante entre un côté inférieur et un côté supérieur du porte-bague d'enfoncement (6, 6a) et de la bague d'enfoncement (8, 8a, 8b) et s'étendent par exemple par rapport à l'axe d'outil (WA) au moins de manière axiale ou de manière sensiblement axiale, de manière préférée également en plus de manière radiale ou de manière sensiblement radiale et/ou de manière tangentielle ou de manière sensiblement tangentielle.

4. Outil de fermeture selon la revendication 2 ou 3, **caractérisé en ce que** le logement de bague d'enfoncement ou l'ouverture (7, 7a) formant celui-ci sont profilés au niveau de leur face intérieure et/ou la bague d'enfoncement (8, 8a, 8b) est profilée au niveau de la face périphérique et/ou frontale prenant appui au niveau du porte-bague d'enfoncement (6, 6a), et ce à savoir respectivement pour réaliser une pluralité de zones en relief et de renfoncements ou de creux qui jouxtent, entourant par exemple également au moins en partie lesdites zones en relief, et que les zones en relief forment en cumulé la face de contact du logement de bague d'enfoncement et/ou la face de contact de la bague d'enfoncement (8, 8a, 8b).

5. Outil de fermeture selon la revendication 4, **caractérisé en ce que** la dimension de face de la somme des surfaces de contact ou d'appui formées par les zones en relief pour la bague d'enfoncement (8, 8a, 8b) est nettement plus petite que la dimension de face que des faces de contact ou d'appui non profilées présenteraient en cumulé pour la bague d'enfoncement (8, 8a, 8b), par exemple d'au moins 50 %.

6. Outil de fermeture selon la revendication 4 ou 5, **caractérisé en ce que** le profilage est réalisé sous la forme de rainures (13, 16) et d'entretoises (14, 17, 21) intercalées formant les faces de contact ou d'appui.

7. Outil de fermeture selon l'une quelconque des revendications 4 - 6, **caractérisé en ce que** le profilage pour un appui axial par rapport à l'axe d'outil (WA) de la bague d'enfoncement (8, 8a, 8b) est formé par une pluralité de parties faisant saillie (15, 22), qui sont espacées les unes des autres et sont prévues dans le logement de bague d'enfoncement ou l'ouverture (7, 7a) et/ou au niveau d'une face frontale de la bague d'enfoncement (8b) de manière répartie autour de l'axe d'outil (WA) ou de l'axe (RA) de la bague d'enfoncement (8b).

8. Outil de fermeture selon l'une quelconque des revendications 6 ou 7, **caractérisé en ce que** les rainures (13, 20) formant le profilage sont réalisées avec leur extension longitudinale de manière parallèle ou de manière sensiblement parallèle à l'axe d'outil (WA) ou à un axe (RA) de la bague d'enfoncement (8a, 8b) ou de manière oblique ou sous une forme hélicoïdale par rapport auxdits axes (WA, RA).

9. Outil de fermeture selon l'une quelconque des revendications 6 - 8, **caractérisé en ce que** sont prévus, dans les entretoises (14), des renfoncements ou des rainures (16) supplémentaires, qui débouchent respectivement au niveau des deux extrémités dans une rainure (13) espaçant deux entretoises (14) et sont orientés de manière tangentielle ou de manière sensiblement tangentielle par exemple par rapport à l'axe de machine (WA) ou par rapport à l'axe (RA) de la bague d'enfoncement (8, 8a, 8b).

10. Outil de fermeture selon l'une quelconque des revendications 6 - 9, **caractérisé en ce que** les parties faisant saillie (15) servant d'appui axial de la bague d'enfoncement (8, 8a, 8b) sont réalisées au niveau d'une entretoise (14) respectivement et deviennent de préférence une partie faisant saillie (18) commune, qui est réalisée de manière à présenter une forme annulaire et qui parvient, en entourant l'axe d'outil (WA), dans le logement de bague d'enfoncement ou dans l'ouverture (7a) formant ledit logement.

11. Outil de fermeture selon l'une quelconque des revendications précédentes, **caractérisé en ce qu'**est prévu, dans le porte-bague d'enfoncement (6a), au moins un canal (29, 30) débouchant dans la fente entre le porte-bague d'enfoncement (6a) et des faces adjacentes de la bague d'enfoncement (8, 8a, 8b), servant à amener et/ou à évacuer le milieu de traitement, dans lequel l'au moins un canal (29, 30) est ouvert de préférence au niveau d'une face du porte-bague d'enfoncement (6a) à l'extérieur du logement de bague d'enfoncement ou de l'ouverture (7, 7a) formant ledit logement.

12. Outil de fermeture selon la revendication 11, **caractérisé en ce que** l'au moins un canal (29, 30) débouche dans au moins une rainure (23, 24) réalisée dans le logement de bague d'enfoncement ou l'ouverture (7), entourant par exemple au moins en partie l'axe d'outil (WA), dans lequel par exemple sont prévues deux rainures (23, 24) décalées l'une par rapport à l'autre de manière axiale par rapport à l'axe d'outil (WA), dans lesquelles débouche respectivement au moins un canal (29, 30) et qui sont reliées de préférence l'une à l'autre conformément par écoulement.

13. Outil de fermeture selon la revendication 12, **caractérisé en ce que** des entretoises (25, 26, 27) délimitant de manière latérale l'au moins une rainure (23, 24) forment les zones d'appui axiales ainsi que radiales de la bague d'enfoncement, et que ladite zone d'appui présente de manière préférée le profilage.

14. Bague d'enfoncement destinée à être utilisée dans un outil de fermeture selon l'une quelconque des revendications précédentes, **caractérisée en ce que** la bague d'enfoncement (8a, 8b) présente au niveau de sa face périphérique ou extérieure et/ou au niveau d'au moins une face frontale le profilage.
